(19) Europäisches Patentamt / European Patent Office / Office européen des brevets

(11) **EP 4 657 395 A1**

(12) **DEMANDE DE BREVET EUROPEEN**

(43) Date de publication:
**03.12.2025 Bulletin 2025/49**

(21) Numéro de dépôt: **25179762.7**

(22) Date de dépôt: **29.05.2025**

(51) Classification Internationale des Brevets (IPC):
**G06V 20/69** *(2022.01)*    **G06T 7/00** *(2017.01)*
**G06T 7/11** *(2017.01)*    **G06T 7/174** *(2017.01)*
**G06T 7/194** *(2017.01)*

(52) Classification Coopérative des Brevets (CPC):
**G06V 20/698; G06T 7/0012; G06T 7/11;**
**G06T 7/174; G06T 7/194;** G06T 2207/10056;
G06T 2207/30024

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**GE KH LA MA MD TN**

(30) Priorité: **01.06.2024 FR 2405742**

(71) Demandeur: **Commissariat à l'Energie Atomique et aux Energies Alternatives**
**75015 Paris (FR)**

(72) Inventeurs:
• **GODEFROY, Guillaume**
**38054 GRENOBLE CEDEX 09 (FR)**
• **PAVIOLO, Chiara**
**38054 GRENOBLE CEDEX 09 (FR)**

(74) Mandataire: **INNOV-GROUP**
**209 Avenue Berthelot**
**69007 Lyon (FR)**

(54) **PROCÉDÉ DE SEGMENTATION 3D D'UN ÉCHANTILLON**

(57) Procédé de segmentation 3D d'un échantillon, l'échantillon comportant au moins un objet biologique, l'échantillon se développant au cours du temps, de façon qu'au moins un objet biologique se divise ou change de forme ou de position au cours du temps, le procédé compotant :
- en différents instants, acquisition d'une pile d'images *(P(t))* de l'échantillon ;
- segmentation d'images, de façon à obtenir des masques correspond à chaque objet biologique ;
- mise en œuvre d'un algorithme de segmentation, dit prompté, de façon à utiliser des masques obtenus, pour un objet, dans une image, pour définir des masques, pour le même objet, dans une autre image.

**Fig. 2**

## Description

### DOMAINE TECHNIQUE

Le domaine technique de l'invention est l'observation de structures microscopiques 3D, par exemple des cellules ou des embryons en cours de développement.

### ART ANTERIEUR

Dans le domaine de la biologie, il peut être utile d'étudier le développement d'objets cellulaires, comme des embryons. Aux stades précoces du développement, le nombre de cellules dans un embryon se multiplie progressivement, et forme une morula. La morula correspond au nom donné à l'embryon lorsqu'il dénombre au moins 16 cellules. Avant ou durant l'atteinte de l'état de morula, il peut être intéressant de monitorer le développement d'un embryon, à des fins de compréhension de mécanismes régissant la fécondation et les premières divisions cellulaires, ainsi que la différenciation cellulaire. Cela peut également permettre de comprendre les causes d'anomalies du développement, ainsi que d'optimiser des techniques de fécondation in vitro.

Différentes publications décrivent le suivi de la trajectoire ou de l'état de cellules, à partir d'images bidimensionnelles acquises au cours du temps. On peut par exemple citer :

- Copperman J. et al" « Morphological cell state description via live-cell imaging trajectory embedding », Communication Biology vol. 6, n°1, 2023-05-04 ;
- Zargari A. et al. « DeepSea : an efficient deep learning model for automated cell segmentation and tracking", bioRxiv, 2021-03-10 ;
- Leon J. et al. "Learning to segment mouse embryo cells", proceedings of SPIE, vol. 10572, 2017-11-17.

Ces publications sont basées sur une acquisition de séquences temporelles d'images 2D.

Des systèmes d'acquisition ont été développés, permettant une observation 3D d'échantillons biologiques. Un tel système est par exemple décrit dans EP4207077.

Cependant, la configuration d'un objet pluricellulaire, comme un embryon, varie dans le temps. On a besoin d'outils permettant une meilleure visualisation ou d'analyse des cellules, et de leur évolution dans le temps. L'invention décrite par la suite répond à ce besoin.

### EXPOSE DE L'INVENTION

**Un** premier objet de l'invention est un procédé de segmentation 3D d'un échantillon, l'échantillon comportant au moins un objet biologique, l'échantillon se développant au cours du temps, de façon qu'au moins un objet biologique se divise ou change de forme ou de position au cours du temps, le procédé comportant :

- a) en différents instants, acquisition d'une pile d'images de l'échantillon, chaque image de la pile d'images représentant un plan de coupe de l'échantillon, chaque pile d'images représentant l'échantillon à un instant, les instants définissant des plages temporelles, de façon qu'au cours de chaque temporelle, l'échantillon comporte un même nombre d'objets biologiques, au moins une pile d'images étant acquise dans chaque plage temporelle ;
- b) segmentation d'au moins une image d'une pile d'images de façon à définir des masques sur ladite image, chaque masque étant délimité par un contour fermé ;
- c) sélection des masques définis lors de l'étape b) en fonction de critères de sélection prédéfinis, de façon que chaque masque sélectionné corresponde à un objet biologique, chaque masque sélectionné étant associé à une position qui correspond à une position du masque sur l'image segmentée;
- d) réitération des étapes b) et c) sur une autre image d'une pile d'images de la même plage temporelle, jusqu'à obtenir moins un masque sélectionné pour chaque objet biologique de l'échantillon ;
- e) sélection d'une pile d'images, acquise durant une plage temporelle, dite plage temporelle d'intérêt ;
- f) pour au moins un objet biologique, à partir d'au moins un masque, sélectionné dans la plage temporelle d'intérêt, et correspondant à l'objet biologique, segmentation d'au moins une image, et de préférence de chaque image de la pile d'image sélectionnée dans l'étape e), de façon à déterminer, dans plusieurs d'images de ladite pile d'images, des masques correspondant à l'objet biologique, la segmentation étant guidée par la position du masque sélectionné ;
- g) répétition des étapes e) à f) pour différentes piles d'images dans au moins une ou dans chaque plage temporelle.

Les étapes b) à g) sont mises en œuvre par une unité de traitement.

Les étapes e) et f) peuvent être répétées pour chaque pile d'images de chaque plage temporelle. L'étape f) peut être répétée pour chaque objet biologique de l'échantillon, dans au moins une plage temporelle.

Le procédé peut comporter, préalablement à l'étape b), une obtention d'un nombre $N(\Delta t)$ d'objets biologiques contenus dans l'échantillon, au cours de la ou de chaque plage temporelle, $N(\Delta t)$ étant un entier supérieur ou égal à 1.

Le procédé peut comporter, préalablement à l'étape b) une détermination du nombre d'objets biologiques, dans au moins une plage temporelle, à partir d'au moins une pile d'image acquise durant la plage temporelle.

Selon une possibilité :

- les instants d'acquisition sont répartis en différentes plages temporelles, à chaque plage temporelle correspondant un nombre d'objets biologiques dans l'échantillon ;
- le procédé comporte, préalablement à l'étape b), une classification de chaque pile d'images, de façon à assigner chaque pile d'images à une desdites plages temporelles.

Selon une possibilité, le procédé comporte, comportant préalablement à l'étape b) :

- i) application d'un algorithme de réduction de la dimension à chaque pile d'images, de façon à assigner une coordonnée à chaque pile d'images dans un espace latent ;
- ii) dans l'espace latent, assignation de chaque coordonnée dans une classe ;
- iii) détermination du nombre d'objets biologique dans l'échantillon, pour chaque pile d'images, en fonction de la classe correspondant à la pile d'images.

L'étape i) peut comporter une utilisation d'un réseau de neurones encodeur, à partir de chaque pile d'images, pour obtenir un code correspondant à chaque pile d'image, l'algorithme de réduction de la dimension étant appliqué à partir du code.

L''étape i) peut comporter une formation d'une image représentative de chaque pile d'images, pour alimenter le réseau de neurones encodeur.

L'image représentative peut être une image de projection maximale établie pour chaque pile d'images.

Les plans de coupe respectifs de chaque image d'une même pile d'images peuvent être parallèles les uns aux autres, ou, alternativement, inclinés angulairement les uns par rapport aux autres.

Dans l'étape c), au moins un critère de sélection peut être choisi parmi :

- un critère morphologique du masque ;
- un recouvrement maximal entre deux masques sélectionnés.

Le critère morphologique peut être choisi parmi :

- une aire minimale et/ou une aire maximale délimitée par le contour de chaque masque ;
- un critère de forme du contour de chaque masque.

Le critère de forme peut correspondre à une forme circulaire ou une forme ellipsoïdale d'excentricité inférieure à une valeur seuil.

L'étape c) peut comporter une détermination d'une propriété physique de l'échantillon dans une partie de l'image correspondant à chaque masque, le critère de sélection dépendant de la valeur de ladite propriété physique.

La propriété physique peut être une propriété optique de l'échantillon, par exemple un indice de réfraction, ou un déphasage de la lumière induit l'échantillon, ou une absorbance de la lumière par l'échantillon.

Selon une possibilité :

- lors de l'étape a), deux piles d'images successivement acquises sont décalées d'un incrément temporel, deux images adjacentes d'une même pile d'images étant décalées d'un incrément spatial,;
- chaque image, sur laquelle au moins un masque a été sélectionné lors d'une étape c) est une image de référence pour l'objet biologique correspondant au masque ;
- l'étape f) comporte :

  • f-i) sélection d'un objet biologique ;
  • f-ii) sélection d'une image dans laquelle aucun masque n'a été défini pour l'objet biologique sélectionné, l'image sélectionnée comportant une coordonnée spatiale et une coordonnée temporelle ;
  • f-iii) sélection d'un masque correspondant à l'objet biologique sélectionné dans une image de référence, pour l'objet biologique sélectionné dans la sous-étape f-i), voisine de l'image sélectionnée dans la sous-étape f-ii), l'image de référence voisine ayant des coordonnées respectivement spatiale et temporelle décalées respectivement d'un nombre d'incréments spatiaux et temporels inférieur à un seuil prédéterminé ;
  • f-iv) report de la position du masque sélectionné dans la sous-étape f-iii) sur l'image sélectionnée dans la sous-étape f-ii) ;
  • f-v) segmentation de l'image sélectionnée dans la sous-étape f-ii), en utilisant la position reportée dans la sous-étape f-iv), de façon à définir un masque pour l'objet biologique sélectionné dans la sous-étape f-i), la segmentation étant guidée par ladite position du masque.

Suite à l'étape f-v), l'image sélectionnée dans la sous-étape f-ii) peut devenir une image de référence pour l'objet biologique sélectionné dans la sous-étape f-i).

Selon une possibilité :

- dans la sous-étape f-iii), la position du masque sélectionnée est définie par une boite englobante délimitant ledit masque ;
- la sous-étape f-iv) comporte un report de la boite englobante sur l'image sélectionnée dans la sous-étape f-ii).

Les sous-étapes f-i) à f-v) peuvent être mises en œuvre de façon à obtenir un masque pour chaque objet biologique de l'échantillon dans chaque images de chaque pile d'images. L'échantillon peut être un organisme multicellulaire, chaque objet biologique étant une cellule. L'échantillon peut être un embryon, chaque objet biologique étant une cellule.

Un deuxième objet de l'invention est un dispositif d'observation d'un échantillon comportant :

- un système d'acquisition, configuré pour former une pile d'images de l'échantillon en différents instants, chaque pile d'images représentant l'échantillon à un instant, les instants définissant des plages temporelles, au cours desquelles l'échantillon comporte un même nombre d'objets biologiques, de façon qu'à chaque plage temporelle correspond au moins une pile d'images;
- une unité de traitement, configurée pour mettre en œuvre les étapes b) à g) d'un procédé selon le premier objet de l'invention, à partir des piles d'images respectivement formées aux différents instants.

Un troisième objet de l'invention est un support, pouvant être connecté à un ordinateur, comportant des instructions pour mettre en œuvre l'étape b) à g) d'un procédé selon le premier objet de l'invention à partir de piles d'images d'un échantillon.

L'invention sera mieux comprise à la lecture de l'exposé des exemples de réalisation présentés, dans la suite de la description, en lien avec les figures listées ci-dessous.

## FIGURES

La figure 1A schématise un exemple de dispositif selon l'invention.

La figure 1B schématise un autre exemple de dispositif selon l'invention.

La figure 2 illustre une pile d'images.

La figure 3A illustre les principales étapes d'un procédé de segmentation 3D d'un échantillon selon l'invention.

La figure 3B détaille des sous-étapes d'une étape d'obtention d'autant de masques que de cellules sur une pile d'images.

La figure 3C détaille des sous-étapes d'une étape de segmentation des images formant une pile d'images.

La figure 4 schématise une mise en œuvre d'un auto-encodeur.

Les figures 5A et 5D montrent des piles d'images à différents instants respectivement.

Les figures 5B et 5E montrent des images de projection maximales respectivement obtenues à partir des piles d'image représentées sur les figures 5A et 5D.

Les figures 5C et 5F schématisent des codes, respectivement obtenus par l'application d'un auto-encodeur à partir des images de projection maximales représentées sur les figures 5B et 5E.

La figure 6 illustre une projection de piles d'images dans un espace latent de dimension 2, et la répartition de chaque piles d'image selon des clusters (grappes). Sur la figure 6, chaque pile d'images est matérialisée par un point.

La figure 7 illustre une évolution temporelle de la classe associée à chaque pile d'images.

Les figures 8A, 8D et 8G montrent des images d'une même pile d'images.

Les figures 8B, 8E et 8H montrent une partie de masques de segmentation obtenus respectivement à partir des images 8A, 8D et 8G.

Les figures 8C, 8F et 8I montrent des masques sélectionnés, suite aux segmentations respectives des images 8A, 8D et 8G

Les figure 9A montre une image d'une pile d'images.

La figure 9B représente des masques de segmentation obtenus par une segmentation de l'image représentée sur la figure 9A.

Les figures 9C, 9D et 9E montrent des images de référence, sur lesquelles une cellule a été segmentée. Les figures 9C, 9D et 9E correspondent à des images temporellement et/ou spatialement décalées par rapport à l'image de la figure 9A.

Les figures 9F, 9G et 9H montrent des masques, correspondant à un même objet, définis sur les figures 9C, 9D et 9E respectivement.

Les figures 9I, 9J et 9K montrent des masques de la cellule obtenus sur l'image de la figure 9A, à partir des masques respectivement représentés sur les figures 9F, 9G, 9H.

La figure 10 montre une application de la segmentation 3D d'un échantillon, pour visualiser les différentes cellules composant l'échantillon.

## EXPOSE DE MODES DE REALISATION PARTICULIERS

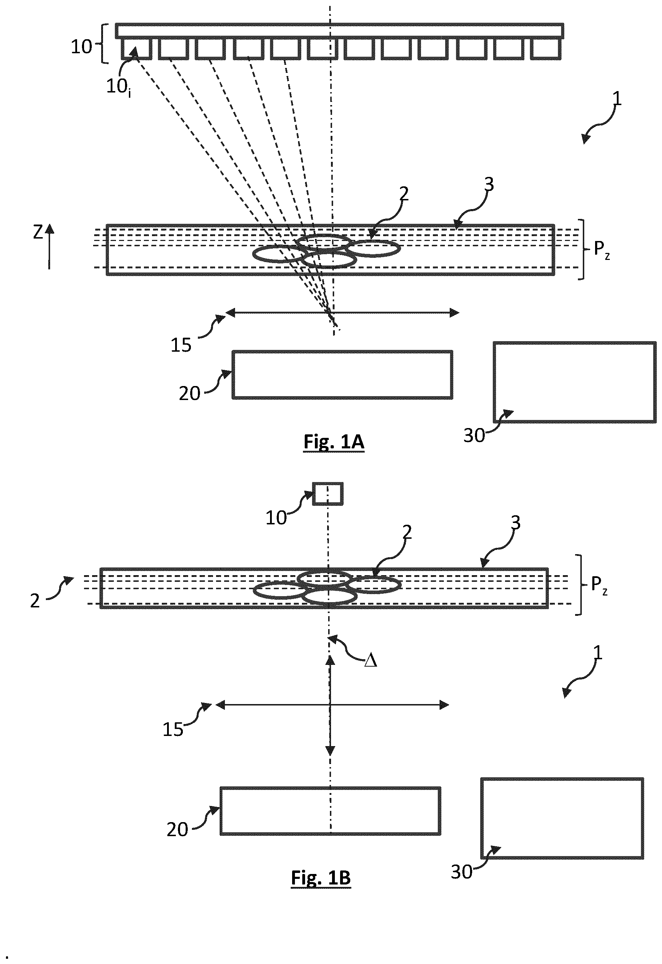

La figure 1A représente un exemple de dispositif 1 permettant une mise en œuvre de l'invention. Le dispositif comporte une source de lumière 11, configurée pour illuminer un échantillon 2. La source de lumière est formée d'une pluralité de sources élémentaires $11_i$, ces dernières étant des diodes électroluminescentes. L'échantillon 2 est un échantillon biologique, dont on souhaite observer le développement. Ainsi, l'échantillon comporte un ou plusieurs objets biologiques. L'échantillon se développe dans le temps, de façon que le nombre et/ou la position et/ou la forme des objets biologiques peuvent évoluer dans le temps. Les objets biologiques peuvent notamment se diviser. A titre d'exemple non limitatif, l'échantillon est échantillon cellulaire, tel un embryon, notamment un embryon non humain. Dans cet exemple l'embryon est un embryon de souris. L'échantillon est contenu dans un récipient 3. L'embryon comporte des cellules, dont le nombre et la forme évoluent dans le temps.

Le dispositif comporte un objectif 15, couplé à un capteur d'image 20. Au cours de la mise en œuvre du dispositif, chaque source de lumière élémentaire $11_i$ est illuminée séquentiellement, et une image de l'échantillon est acquise pour chaque illumination d'une source de lumière élémentaire. Cela permet d'illumination de l'échantillon selon un angle d'incidence variable, une image de l'échantillon étant acquise pour chaque angle d'incidence. A partir des différents images acquises, une unité de traitement 30 permet d'effectuer une reconstruction de l'échantillon, selon des plans de coupe de préférence parallèles les uns aux autres, et orthogonaux à un axe Z. L'unité de traitement 30 comporte au moins un microprocesseur, configuré pour exécuter des instructions mémorisées et permettre la mise en œuvre l'algorithmes. L'unité de traitement 30 met en œuvre un algorithme de reconstruction tomographique. La demande de brevet EP4207077 décrit un dispositif tel que représenté sur la figure 1A ainsi que la mise en œuvre d'un algorithme permettant l'obtention d'images d'absorption ou de phase selon des plans de coupe. Dans cet exemple, on utilise des images de phase de l'échantillon.

La figure 1B représente un autre dispositif permettant une mise en œuvre de l'invention. Le dispositif comporte une source de lumière 11, configurée pour illuminer un échantillon 2. De même que dans la figure 1A, la source de lumière peut être une diode électroluminescente.

Le dispositif comporte un capteur d'image 20 est couplé à un objectif 15, ce dernier permettant de conjuguer un plan objet, dit plan focal, avec le capteur d'image. Le capteur d'image et le système optique sont alignés selon un axe optique Δ. L'ensemble formé par le capteur d'image et le système optique est configuré de façon que le plan focal puisse être translaté parallèlement à l'axe optique Δ, selon différentes profondeurs à l'intérieur de l'échantillon.

De façon alternative, le capteur d'image est associé à un diaphragme confocal, ce dernier permettant l'observation successive de différentes tranches de l'échantillon.

Ainsi, d'une façon générale, on dispose d'un système d'acquisition 1, configuré pour former des images d'un échantillon selon différents plans de coupe, formant une pile d'images. Les images acquises, formant la pile d'images, peuvent être des images standard, des images de l'absorbance ou des images de phase ou es images de diffraction ou des images de fluorescence de l'échantillon, lorsque ce dernier comporte un marqueur fluorescent, ou plus généralement tout type de grandeur mesurable par un microscope.

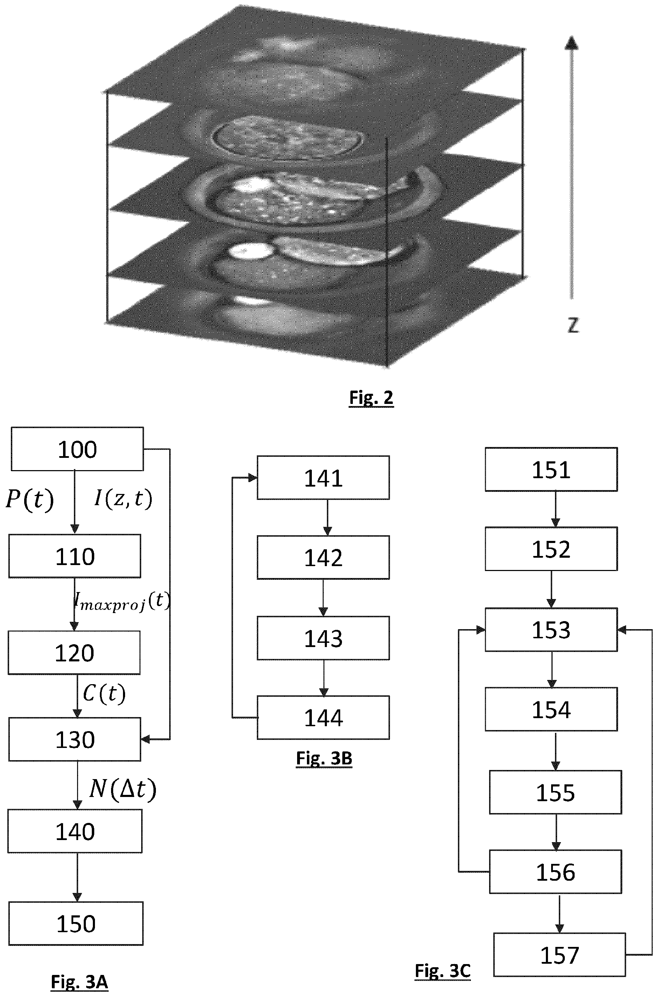

La figure 2 représente un exemple de plans de coupe d'un embryon de souris, obtenu avec un dispositif tel que schématisé sur la figure 1A.

Un objectif de l'invention est de permettre un suivi, dans le temps du développement de l'échantillon, et cela en trois dimensions. Selon un aspect complémentaire, l'invention permet d'effectuer une classification de phases de développement de l'échantillon, à partir de piles d'images acquises en différents instants.

La figure 3A schématise différentes étapes mises en œuvre par l'unité de traitement 30, ou par tout autre moyen approprié.

Etape 100 : acquisition de piles d'images en différents instants.

Au cours de cette étape, plusieurs piles d'images $P(t)$ sont formées, respectivement à différents instants t. Chaque pile d'images est formée à partir d'images acquises, par le capteur d'image, à chaque instant. Comme décrit en lien avec les figures 1A et 1B, à chaque instant, le capteur d'image acquiert une série d'images, qui sont utilisées pour former une pile d'images. Les images de la série d'images sont considérées comme ayant été acquise au même instant, l'écart temporel entre les acquisitions des images d'une même série d'images étant négligé.

Chaque image de la pile d'images correspond soit à une image acquise par le capteur d'image, soit à une image obtenue des traitements, notamment une reconstruction tomographique, à partir d'images de l'échantillon. Chaque pile d'images $P(t)$ comporte des images $I(z, t)$. L'indice z correspond à un index spatial de chaque image, selon l'axe Z, avec $z_0 \leq z \leq z_{max}$. L'indice t est un index temporel, relatif aux instants d'acquisition. Les instants s'étendent entre un instant initial $t_0$ et un instant final $t_f$. La période s'écoulant entre $t_0$ et $t_f$ est la période d'acquisition T. Les différentes piles d'images permettent d'obtenir des informations quant au développement spatio-temporel de l'échantillon.

La période d'acquisition T comporte une ou plusieurs plages temporelles Δt. Au cours de chaque plage temporelle, on considère que l'échantillon comporte un même nombre d'objets biologiques N(Δt), en l'occurrence un même nombre de cellules. Les plages temporelles, ainsi que le nombre d'objets biologiques pour chaque plage temporelle, peuvent être connus, par exemple à partir d'un a priori ou d'une autre méthode de mesure. Alternativement, et optionnellement, les plages temporelles ainsi que le nombre d'objets pour chaque plage temporelle peuvent être déterminés à partir des piles d'images, comme décrit en lien avec les étapes 110 à 130.

A des fins de clarification, le terme « série d'images » désigne des images acquises par le capteur, à partir desquelles on obtient une pile d'images, qui correspond à des images de l'échantillon selon différents plans, et de préférence selon différents plans parallèles les uns aux autres. A chaque instant, on acquiert une série d'images, à partir de laquelle une pile d'image est obtenue. Le nombre de plans de coupe peut être de quelques dizaines à quelques milliers. Dans l'exemple représenté, on a pris en compte entre 50 et 150 plans de coupe.

Selon une possibilité, les plans de coupe sont angulairement espacés les uns des autres.

Chaque pile d'images dépend de l'état d'un échantillon biologique à un instant t. Les étapes 110 à 130, décrites ci-dessous, permettent un suivi temporel de l'état de l'échantillon cellulaire, en passant par une réduction de la dimension (étapes 110 à 120) et une classification (étape 130).

Etape 110 : compression.

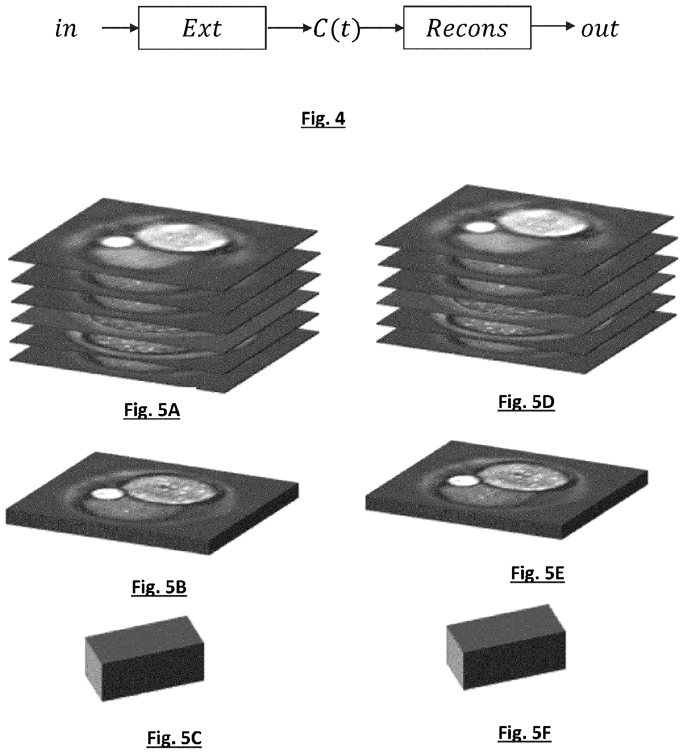

Au cours de la période d'acquisition, l'échantillon cellulaire se développe. A partir des piles d'images *P(t),* on met en œuvre un algorithme de classification, pour identifier différentes phases du développement de l'échantillon : il s'agit d'identifier les plages temporelles Pour ce faire, on utilise un code C(t) de chaque pile d'images *P(t),* de dimension réduite par rapport à chaque pile d'image, et porteuse d'information concernant. Un code d'une pile d'images peut être obtenu en mettant en œuvre un réseau de neurones convolutif de type encodeur. Il peut par exemple s'agir de l'encodeur d'un auto-encodeur tel que schématisé sur la figure 4. De façon connue de l'homme du métier, un réseau de neurones de type auto-encodeur est une structure comportant un bloc d'extraction *Ext,* dit encodeur, permettant d'extraire une information pertinente à partir d'une donnée d'entrée *In,* généralement de grande dimension, définie dans un espace de départ. L'information extraite par le bloc d'extraction est appelée code *C(t).* L'auto-encodeur comporte un bloc de reconstruction *Recons* permettant une reconstruction du code, de façon à obtenir une donnée de sortie *out,* définie dans un espace généralement identique à l'espace de départ. L'apprentissage de l'auto-encodeur est effectué de façon à minimiser une erreur entre la donnée d'entrée *in* et la donnée de sortie *out.* Suite à l'apprentissage, le code, extrait par le bloc d'extraction, est considéré comme représentatif des caractéristiques principales de la donnée d'entrée. Autrement dit, le bloc d'extraction permet une compression de l'information contenue dans la donnée d'entrée.

Selon une possibilité, la sortie de l'auto-encodeur n'est pas l'image fournie en entrée, mais un masque représentatifs des objets représentés sur l'image d'entrée.

La donnée d'entrée peut être une pile d'images. Cependant, il est préférable de former une donnée d'entrée comportant une information compressée de la pile d'images. La donnée d'entrée de l'encodeur peut être une image, dite image de projection maximale $I_{maxproj}(t)$. Chaque image $I(z, t)$ est définie selon des pixels r. En chaque pixel r, l'image de projection maximale est telle que

$$I_{maxproj}(t,r) = \max_{z_0 \leq z \leq z_{max}} I(z,t,r) \ (1)$$

A chaque instant *t,* à partir d'une pile d'image *P(t),* on peut obtenir une image de projection maximale $I_{maxproj}(t)$, qui forme la donnée d'entrée l'encodeur. Le code *C(t)* généré par l'encodeur comporte l'information relative à la pile d'image *P(t).*

Sur les figures 5A, 5B et 5C, on a respectivement schématisé une pile d'image *P(t),* une image de projection maximale $I_{maxproj}(t)$ et un code *C(t)* à un instant $t = t_1$.

Sur les figures 5D, 5E et 5F, on a respectivement schématisé une pile d'image *P(t),* une image de projection maximale $I_{maxproj}(t)$ et code *C(t)* à un instant $t = t_2$.

Dans cet exemple, le code est un bloc de données de dimensions 254 x 254 x 16

Le recours à une image de projection maximale pour alimenter l'encodeur n'est pas une condition nécessaire. L'encodeur peut être alimenté par une ou quelques images de la pile d'images. L'encodeur peut également être alimenté par une autre transformation des images de la pile d'images, par exemple une image moyenne ou une image médiane de chaque image de la pile d'images.

Etape 120 : Réduction de dimension

Au cours de cette étape, chaque code *C(t)* fait l'objet d'une réduction de dimension, en étant projetée dans un espace latent *L* de dimension moindre que le nombre de termes formant le code *C(t).* La dimension de l'espace latent est de préférence inférieure ou égale à 5, de préférence inférieure ou égale à 3, par exemple égale à 2.

Différentes méthodes de réduction de dimensions sont disponibles. D'une façon générale, une méthode de réduction de dimensionnalité permet de représenter des coordonnées d'origine, dans un espace de départ, de dimension N, en des coordonnées dans un espace latent de dimension *M,* avec *M < N.* La dimension de l'espace latent dépend de la dimension de l'espace de départ et de leur complexité. Le passage de l'espace de départ vers l'espace latent est effectué par une fonction de projection *f* appliquée à un vecteur comportant l'ensemble des termes formant le code.

Plusieurs méthodes de réduction de dimensions sont connues de l'homme du métier. Par exemple, l'analyse en composante principales (ACP) est une méthode non supervisée et linéaire de réduction de dimensionnalité. Des méthodes non linéaires peuvent être mises en œuvre, par exemple la méthode UMAP (Uniform Manifold Approximation and Projection).

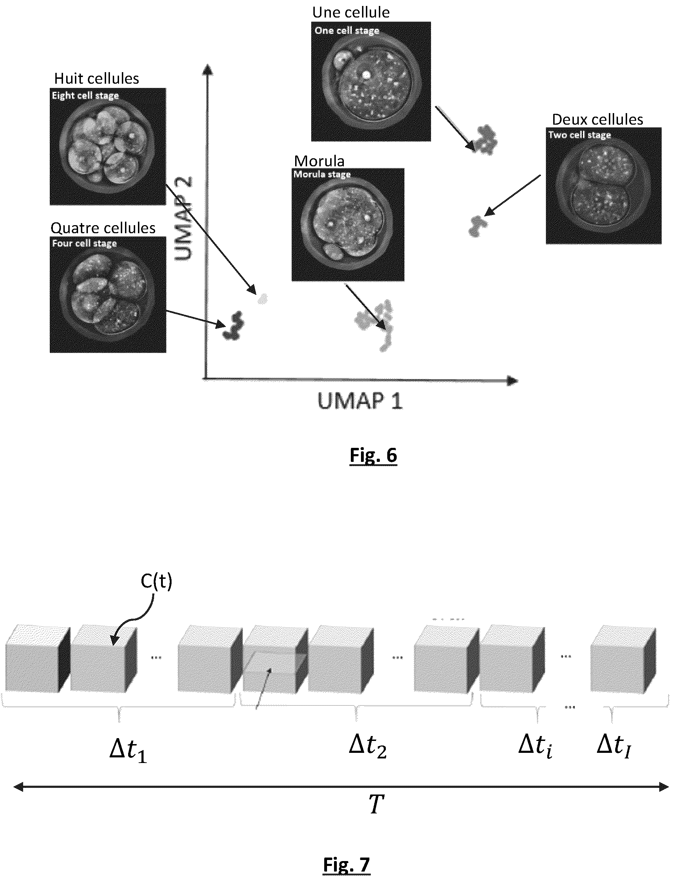

Sur la figure 6, on a représenté différentes projections de code *C(t)* correspondant respectivement à différentes piles d'images *P(t).* L'espace latent est défini dans une base UMAP1, UMAP2 à deux dimensions. Sur la figure 6, chaque point correspond à un code *C(t),* ce dernier correspondant à un pile d'images *P(t).*

Dans cet exemple, l'algorithme de réduction de dimensions est effectué à partir d'une information compressée, qui correspond à l'image de projection maximale. Le recours à l'image de projection maximale est avantageux lorsque le nombre de plans de coupe est élevé. Cependant, l'algorithme de réduction de dimensions peut être mis en œuvre directement à partir des piles d'images, les images de la pile d'image formant les données d'entrée de l'encodeur. C'est notamment le cas lorsqu'on dispose d'un nombre réduit de plans de coupes.

L'étape 120 de réduction de dimension est optionnelle.

Etape 130 : Classification

Au cours de cette étape, on met en œuvre un algorithme de classification, de façon à assigner une classe à chaque point, dans l'espace latent L, chaque point correspondant à une pile d'images, c'est-à-dire à l'état de l'échantillon à un instant t. Une classe est ainsi assignée à l'échantillon, à chaque instant. Les classes peuvent être prédéfinies ou non. On peut par exemple mettre en œuvre un algorithme de classification non supervisé, par exemple Kmeans. Le recours à d'autres algorithmes de classification est envisageable, par exemple Kmeans, Birch (Balance iterative reducing and clustering using hierarchies) ou GMM (Gaussian Mixture Models) Chaque classe est représentative d'un état de l'échantillon, au cours d'instants successifs dans une période temporelle $\Delta t$ appartenant à la période d'acquisition T.

Sur la figure 6, on a représenté 5 clusters (grappes), formant 5 classes. Au cours de la période d'acquisition, l'échantillon est successivement assigné à chacune de ces classes, en fonction de son développement. Chaque classe correspond à une plage temporelle $\Delta t$ au cours de laquelle le nombre de cellules, dans l'échantillon, est constant. Par exemple, lorsque l'échantillon est un embryon, on peut identifier cinq phases temporelles du développement, en fonction du nombre de cellules comprises dans l'embryon : une première classe correspond à une cellule unique. Une deuxième classe correspond à 2 cellules. Les troisièmes, quatrièmes correspondent respectivement à 4 et 8 cellules. La cinquième classe correspond à la formation d'un blastocyte. Sur la figure 6, on a illustré les différentes phases de développement de l'échantillon correspondant respectivement à chaque classe.

Ainsi, chaque classe est représentative d'un nombre de cellules dans l'échantillon ou d'un état de l'échantillon. L'assignation d'un échantillon à chaque classe permet ainsi de déterminer le nombre de cellules $N(\Delta t)$ dans l'échantillon à chaque instant d'acquisition.

La figure 7 montre l'évolution temporelle de la classification de l'échantillon. Sur la figure 7, on a représenté des piles d'images P(t) regroupées par classes, chaque classe correspondant à une plage temporelle $\Delta t_i$. $i$ est l'index de chaque période temporelle, compris entre 1 et $l$, $l$ correspondant au nombre de classes.

De préférence, la classification est une classification non supervisée, usuellement désignée par le terme anglosaxon clustering.

Selon une possibilité le clustering est effectué directement à partir de chaque pile d'images ou à partir d'une image obtenue par traitement de chaque pile d'images, par exemple à partir d'une image de projection maximale, ou à partir d'un code résultant d'un encodeur. La classification peut être effectuée par un réseau de neurones ou un autre algorithme de clustering.

Etape 140 : segmentation - définition de masques

Cette étape est mise en œuvre sur chaque pile d'images. Au cours de cette étape, certaines images de chaque pile d'images font l'objet d'une segmentation, afin de définir un masque. Un masque est une zone d'une image de coupe correspondant à un même objet, par exemple une cellule.

Dans cet exemple, une des données d'entrée des images est le nombre de cellules $N(\Delta t)$ à segmenter. Ce nombre peut être défini sur la base d'un a priori, ou d'une autre modalité de mesure, auquel cas les étapes 110 à 130 ne sont pas nécessaires. Lorsque les étapes 110 à 130 sont mises en œuvre, le nombre de cellules $N(\Delta t)$ à segmenter dépend de la classe assignée à la pile d'images.

Une autre donnée d'entrée est un a-priori quant à la morphologie et/ou la disposition de chaque masque. Ainsi, la segmentation est effectuée en prenant en compte des contraintes concernant la géométrie du masque : forme géométrique du contour, absence de « trous » dans le masque, taux de recouvrement maximal entre deux masques adjacents, voire absence de recouvrement entre deux masques adjacents. Dans le cas de cellules, les contraintes morphologiques peuvent être :

- un contour de forme ellipsoïdale ou circulaire, dont l'excentricité est supérieure à une valeur seuil, ou comprise dans une plage de valeurs prédéterminées ;
- une aire comprise entre une aire minimale et une aire maximale ;
- un taux de recouvrement nul ou inférieur à 10% par exemple.

D'une façon générale, une contrainte morphologique permet de définir une correspondance morphologique avec un modèle de masque, correspondant à un objet biologique recherché.

D'autres types de contraintes peuvent être envisagées, par exemple des contraintes relatives à des grandeurs physiques estimées à partir de chaque image. Il peut s'agir d'une valeur ou d'une plage de valeurs d'indices de réfraction ou de déphasage.

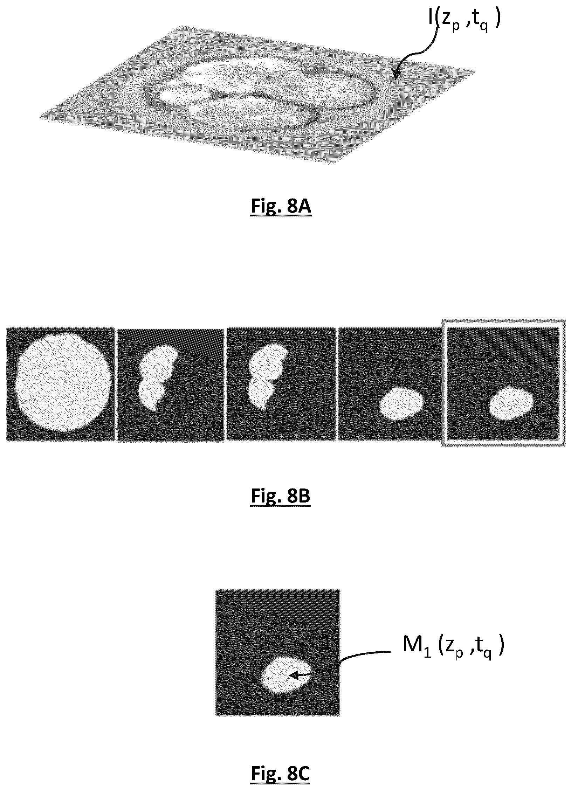

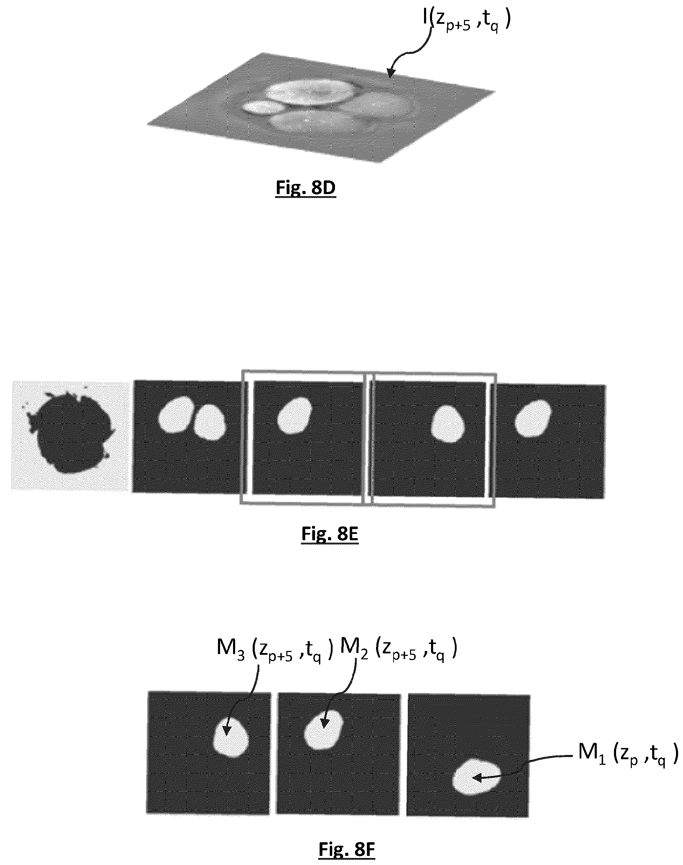

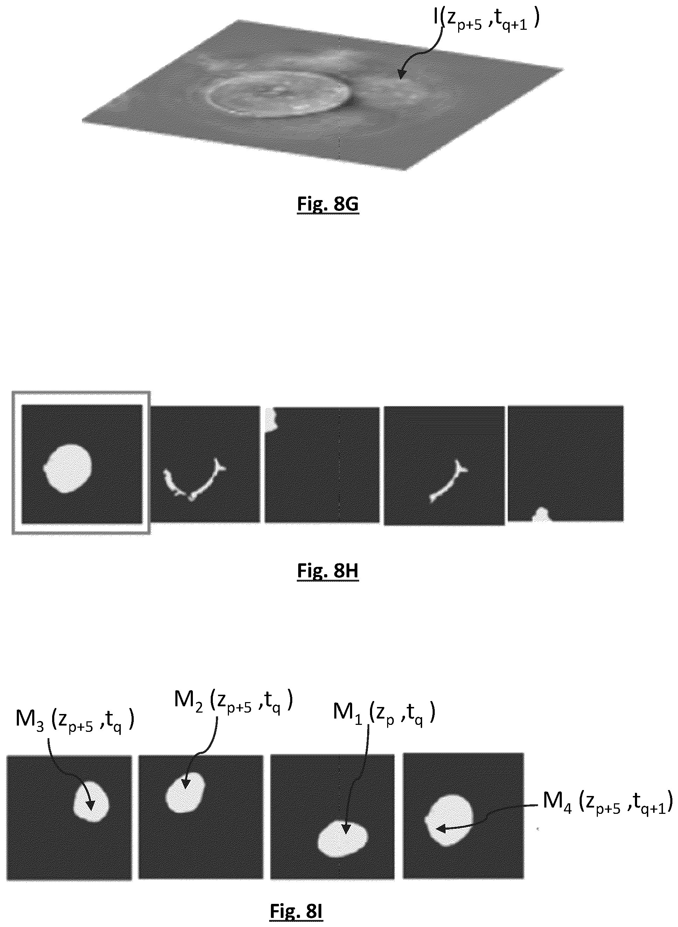

Au cours de cette étape, on effectue une segmentation de chaque image, jusqu'à l'identification d'un nombre de masques correspondant au nombre de cellules $N(\Delta t)$ sur l'image. Les figures 8A à 8I illustrent le procédé de segmentation et de définition de masques sur une pile d'images. Dans cet exemple, on pris une pile d'images classifiées dans la classe correspondant à un nombre de cellules égales à 4. On prend en compte une première image $I(z_p,t_q)$, qui peut être choisie arbitrairement. p désigne un indice de coordonnées spatiales et q désigne un indice de coordonnées temporelles. Il peut par exemple s'agir d'une image dans un plan médian de l'échantillon. L'image $I(z_p,t_q)$ est représentée sur la figure 8A. L'algorithme de segmentation permet de définir des masques, dont cinq sont représentés sur la figure 8B. L'algorithme de segmentation est par exemple l'algorithme de segmentation d'images bidimensionnelles SAM « Segment Anything Model », décrit dans Cen, J « Segment Anything in 3D with NerFs ». En général, le nombre de masques différents identifiés par l'algorithme peut être supérieur à 10, voire à plusieurs dizaines. Sur la figure 8B, on a représenté que cinq masques à titre d'exemple. Idem pour les figures 8E et 8H décrites par la suite.

Les masques identifiés font l'objet d'un filtrage, prenant en compte les contraintes morphologiques. Seuls les masques satisfaisant aux contraintes sont sélectionnés, les autres masques étant rejetés. La figure 8C représente un masque $M_1(z_p,t_q)$ sélectionné parmi les masques identifiés sur la figure 8B. Par la suite, chaque masque est désigné $M_u(z_p,t_q)$, l'indice u désignant l'objet correspondant au masque. Dans cet exemple, u est un entier compris entre 1 et 4 car il y a quatre objets biologiques (quatre cellules dans l'échantillon). $M_u(z_p,t_q)$ est le masque, correspondant à un objet u, obtenu par l'image $I(z_p,t_q)$.

Selon une possibilité, le filtrage comporte une mise en œuvre d'un algorithme d'intelligence artificielle à apprentissage supervisé, par exemple un réseau de neurones. Le réseau de neurones peut alors déterminer si un masque résultant de la segmentation correspond à un objet biologique susceptible d'être présent dans l'objet.

Suite à la segmentation et au filtrage effectués à partir de la première image, un seul masque a été sélectionné (cf. masque $M_1(z_p,t_q)$ sur la figure 8C). Une deuxième image, de la même pile d'images, ou d'une pile d'images de la même plage temporelle, est prise en compte. Le processus de segmentation / filtrage est renouvelé, de façon à identifier un masque différent de celui précédemment sélectionné. La nouvelle image prise en compte appartient à la même plage temporelle $\Delta t$. Elle est décalée spatialement et/ou temporellement par rapport à l'image initiale, d'un ou plusieurs incréments spatiaux et/ou temporels. La figure 8D représente l'image prise en compte : il s'agit de l'image $I(z_{p+5},t_q)$. On met en œuvre l'algorithme de segmentation sur cette image, ce qui permet l'identification de masques, dont six sont représentés sur la figure 8E. Le filtrage permet de sélectionner deux masques, ces derniers étant encadrés sur la figure 8E. Les masques sélectionnés sont notés $M_2(z_{p+5},t_q)$ et $M_3(z_{p+5},t_q)$ car ils correspondent respectivement à la deuxième et à la troisième cellule. Suite à l'analyse de la deuxième image $I(z_{p+5},t_q)$, on dispose de trois masques, adressant respectivement trois objets différents : le masque $M_1(z_p,t_q)$ sélectionné sur la première image $I(z_p,t_q)$ et les masques $M_2(z_{p+5},t_q)$ et $M_3(z_{p+5},t_q)$ sélectionnés sur la deuxième image $I(z_{p+5},t_q)$: ces masques sont représentés sur la figure 8F.

Aucun masque n'a été sélectionné pour le quatrième objet. Une troisième image $I(z_{p+5},t_{q+1})$ est prise en compte : cf. figure 8G. La troisième image fait l'objet d'une segmentation, de façon à identifier des masques : cf. figure 8H. Parmi les masques identifiés, un seul masque est sélectionné, qui correspond au masque encadré sur la figure 8H. Le masque sélectionné $M_4(z_{p+5},t_{q+1})$ est ajouté aux masques sélectionnés au cours des itérations précédentes : cf. figure 8I. On dispose ainsi d'un ensemble de masques, pour la pile d'images, correspondant au nombre de cellules composant l'échantillon cellulaire.

L'étape d'identification et de sélection des masques est ainsi une étape itérative, comportant les sous-étapes suivantes, représentées sur la figure 3B.

- prise en compte d'une k$^{\text{ième}}$ image de la pile d'images, ou d'une pile d'images acquise la même plage temporelle, de façon que chaque image prise en compte représente un même nombre de cellules dans l'échantillon. k désigne le rang de l'itération ; (sous-étape 141) . k est un entier supérieur ou égal à 1. De préférence, la k$^{\text{ième}}$ image est choisie dans une pile d'images temporellement voisine, c'est-à-dire une pile d'images décalée temporellement d'un nombre d'incréments temporels et/ou spatiaux inférieurs à un seuil prédéterminée par rapport à la pile d'images considérée. La valeur du seuil dépend de l'écart spatial entre deux images d'une même pile d'image, et/ou de l'écart temporel entre deux piles d'images successivement acquises. Chaque seuil (spatial ou temporel) est défini au cas par cas.
- segmentation de l'image prise en compte, de façon à identifier des masques (sous-étape 142) ;
- filtrage des masques identifiés, en prenant en compte le ou les critères prédéfinis (sous-étape 143) ;
- réitération des sous-étapes 141 à 143 , jusqu'à ce que le nombre de masques identifiés au cours de chaque itération atteigne le nombre $N(\Delta t)$ de cellules dans l'échantillon (sous-étape 144). D'une façon plus générale, les sous-étapes 141 à 143 sont réitérées jusqu'à obtenir au moins un masque respectivement pour chaque cellule : soit un seul masque pour chaque cellule, soit au moins un masque pour chaque cellule.

On observe que la segmentation n'est pas réalisée sur l'ensemble des images de la pile d'images, mais sur un nombre d'images suffisant de façon à atteindre un nombre de masques suffisants, ne se recouvrant pas (ou partiellement, selon un taux de recouvrement prédéfini).

L'idée est de ne pas obtenir un masque pour chaque cellule sur l'ensemble des images de toutes les piles d'images, mais d'un nombre suffisant de masques, avec au moins un masque par cellule, de façon à pouvoir mettre en œuvre l'étape 150 de segmentation promptée. Par nombre suffisant de masques, on entend 1 seul masque par cellules, on un nombre de masques relativement limité, par exemple moins de 5 ou moins de 10 ou moins de 20 masques par cellule. L'objectif est de disposer d'une définition initiale de masques pour chaque objet, pour pouvoir mettre en œuvre l'algorithme de segmentation promptée décrit en lien avec l'étape 150, ce dernier étant plus performant.

Selon une possibilité, le nombre $N(\Delta t)$ d'objets biologiques dans l'échantillon, lors de chaque plage temporelle, n'est pas connu. Dans ce cas, les sous-étapes 141 à 144 sont mises en œuvre de façon à sélectionner des masques, correspondant à des objets différents jusqu'à ce qu'on ne puisse pas définir un nouveau masque correspondant à un nouvel objet. C'est l'algorithme de segmentation/filtrage qui permet, indirectement, d'obtenir le nombre d'objets biologiques dans l'échantillon. Le nombre d'objets biologiques différents correspond au nombre masques respectivement associés à des objets différents. L'association de chaque masque à un objet est effectuée en fonction de la position de chaque masque sur une image.

A l'issue de l'étape 140, on dispose de préférence, dans chaque pile d'images, d'au moins un masque pour chaque objet biologique. A chaque masque résultant de cette étape est associé une position, qui correspond à la position du masque dans l'image sur laquelle le masque a été défini. Chaque masque sélectionné lors de l'étape 140 peut être considéré comme un masque de base, pour une cellule. La dénomination masque de base signifie que le masque est destiné à être utilisé par la suite, pour définir d'autres masques pour ladite cellule, dans d'autres images acquises durant la même plage temporelle.

Le nombre de masques de base, défini pour une même plage temporelle est limité : il est bien inférieur au nombre d'images acquise dans la plage temporelle multiplié le nombre de cellules formant l'échantillon. Il est par exemple inférieur à 2 fois ou 10 fois, ou 20 fois, ou 50 fois, ou 100 fois inférieur au nombre d'images acquise dans la plage temporelle multiplié par le nombre d'objets.

Etape 150 : propagation - segmentation de l'ensemble de la pile d'images

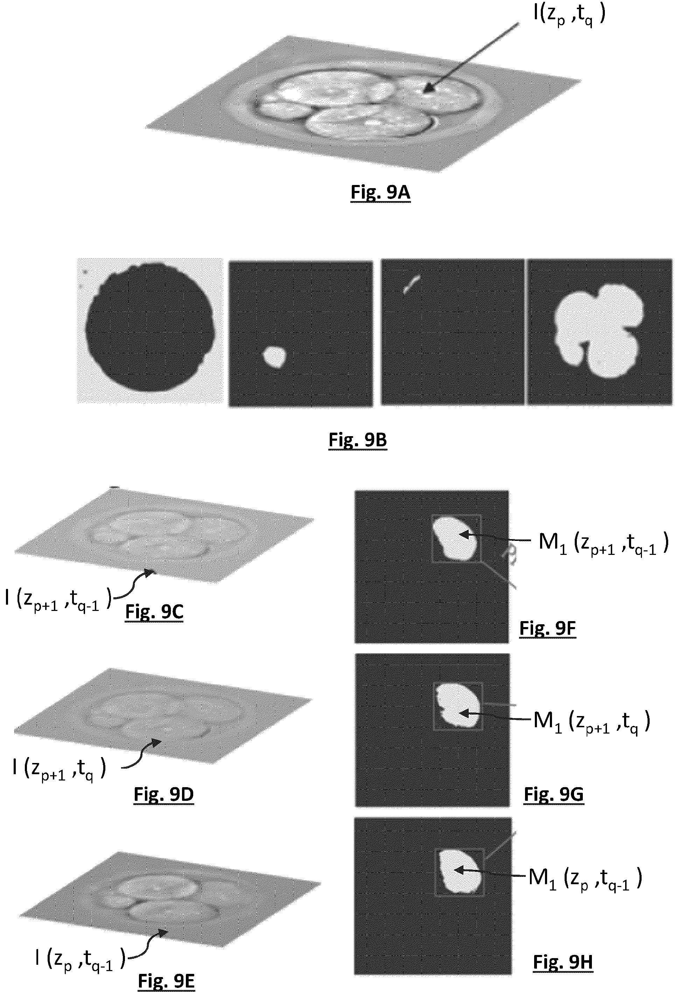

L'étape 150 est mise en œuvre sur chaque pile d'images. Les masques identifiés sur les images prises en compte dans l'étape 140 sont propagés, de proche en proche, sur une même pile d'images, de façon à obtenir une segmentation de chaque image de la pile d'images avec un nombre $N(\Delta t)$ de masques, $N(\Delta t)$ correspondant au nombre de cellules dans l'échantillon. L'étape 150 est illustrée sur les figures 9A à 9H.

La figure 9A représente une image $I(z_p, t_q)$, issue d'une pile d'images.

Il serait possible d'effectuer une segmentation et un filtrage de l'ensemble des images de chaque pile d'images, la segmentation et le filtrage étant effectués indépendamment sur chaque image. Cependant, on a constaté qu'une telle solution, qui semble évidente, engendrait des erreurs. La figure 9B montre par exemple une segmentation d'une image d'une pile d'images, effectuée de façon indépendante des autres images de la même pile d'images. On observe que le résultat n'est pas satisfaisant.

Ayant constaté cela, les inventeurs proposent une méthode de segmentation, dite promptée (ou guidée), selon laquelle on utilise une information initiale résultant d'une segmentation, telle que décrite dans l'étape 140, effectuée :

- dans une image décalée temporellement d'un ou de plusieurs incréments temporels, le nombre d'incréments temporels étant inférieur à un certain seuil de façon que l'image décalée soit pouvant être considérée comme suffisamment « temporellement » proche de l'image traitée ;
- et/ou dans une image décalée spatialement, d'un ou de plusieurs incréments spatiaux, le nombre d'incréments spatiaux étant inférieur à un certain seuil de façon que l'image décalée soit pouvant être considérée comme spatialement suffisamment proche de l'image traitée

L'idée sous-jacente est d'effectuer, sur les images d'une pile d'images, une segmentation promptée, guidée par la position de masques de segmentation identifiés sur des images acquises durant la même plage temporelle $\Delta t$, et de préférence temporellement ou spatialement voisines. La qualité des masques générés par l'algorithme de segmentation promptée est largement supérieure à celle de masques générés par l'algorithme de segmentation sans promptage (i-e sans guidage).

Pour chacune des cellules composant l'échantillon cellulaire, la segmentation des images d'une pile d'images est effectuée progressivement, à partir d'images de référence. Par image de référence, on entend une image de la pile d'image sur laquelle la cellule prise en compte a été segmentée.

L'étape 150 comporte les sous étapes suivantes :

- Sous-étape 151 : sélection d'une pile d'images, appartenant à une plage temporelle $\Delta t$ dans laquelle l'échantillon comporte $N(\Delta t)$ cellules.

- Sous-étape 152 : sélection d'une cellule parmi les $N(\Delta t)$ cellules que contient l'échantillon ;

- Sous étape 153 : sélection d'une image de référence, dans laquelle le masque de la cellule a été identifié. L'image de référence peut être une image de la pile d'images, ou une image d'une pile d'images temporellement décalée de la pile d'image considérée, et appartenant à la même plage temporelle Δt. L'image de référence est de préférence temporellement voisine de la pile d'images, en étant distante de 5 incréments temporels, par exemple, de la pile d'images sélectionnée lors de la sous-étape 151.

- Sous étape 154 : sélection d'une image à analyser dans la pile d'images considérée. De préférence, l'image analysée est formée dans un plan de coupe voisin du plan de coupe sur lequel est formée l'image de référence. Par plan de coupe voisin, on entend un plan de coupe dont la coordonnée, selon l'axe Z, est décalée de un incrément spatial ou quelques incréments spatiaux. Le décalage spatial est par exemple inférieur à 5 incréments spatiaux

- Sous-étape 155 : à partir de la position du masque dans l'image de référence, segmentation de l'image sélectionnée lors de la sous-étape 154. Une façon de déterminer la position du masque dans l'image de référence est de définir une bounding box, ou boite englobante, dans l'image de référence. La bounding box délimite le masque. La bounding box définie dans l'image de référence est reportée dans l'image analysée. L'algorithme de segmentation prend en compte la position de la bounding box dans l'image analysée pour identifier un masque. Ainsi, l'algorithme de segmentation est guidé, ou « prompté » par le masque préalablement défini sur l'image de référence. Cela suppose le recours à un algorithme de segmentation promptable, la segmentation dans une image étant effectuée à partir d'une indication quant à la position probable du masque : cette dernière peut être définie par une bounding box, ou par le centre du masque ou par un nuage de points.
La sous-étape 155 peut être mise en œuvre successivement en prenant en compte plusieurs images de référence, sur lesquelles une même cellule a été délimitée respectivement par différents masques de segmentation. Chaque image de référence est de préférence située dans un voisinage spatial ou temporel de l'image analysée. Dans ce cas, le masque, correspondant à la cellule sélectionnée lors de la sous-étape 152, est reporté, à partir de chaque image de référence, sur l'image analysée. On dispose d'autant de reports de bounding box que d'images de référence prises en compte. L'algorithme de segmentation permet de déterminer autant de masques de segmentation que de bounding boxes reportées. Une étape de filtrage permet de sélectionner, parmi les différents masques de segmentation, le masque de segmentation le plus approprié. Le filtrage est effectué comme en lien avec l'étape 140. Après la sous-étape étape 155, l'image analysée devient une image de référence pour la cellule sélectionnée lors de la sous-étape 152. Elle peut être utilisée, à son tour, pour guider une segmentation d'une autre image pour cette cellule.

- Sous-étape 156 : sélection d'une autre cellule. Les sous-étapes 153 à 155 sont réitérées pour chacune des $N(\Delta t)$ cellules que contient l'échantillon durant la plage temporelle Δt.

- Sous-étape 157 : sélection d'une autre pile d'images dans la même plage temporelle Δt. Après que l'ensemble des images d'une même pile d'image a fait l'objet d'une segmentation, pour l'ensemble des $N(\Delta t)$ cellules, les sous-étapes 153 à 156 sont réitérées pour une autre pile d'images.

L'étape 150 peut être mise en œuvre pour une ou pour plusieurs plages temporelles Δt. L'étape 150 peut être mise en œuvre pour tout ou partie des $N(\Delta t)$ cellules dans chaque plage temporelle.

Les figures 9C, 9D et 9E représentent respectivement, pour une première cellule :

- une image de référence $I(z_{p+1}, t_{q-1})$, appartenant à une pile d'image $P(t_{q-1})$ ;
- une image de référence $I(z_{p+1}, t_q)$, appartenant à une pile d'image $P(t_q)$ ;
- une image de référence $I(z_p, t_{q-1})$, appartenant à une pile d'image $P(t_{q-1})$.

Les images représentées sur les figures 9C, 9D et 9E sont utilisées en tant qu'images de référence pour segmenter une image $I(z_p, t_q)$ afin d'identifier un masque de segmentation correspondant à la première cellule. Sur les figures 9F, 9G et 9H, on a représenté les masques de segmentation $M_1(z_{p+1}, t_{q-1})$, $M_1(z_{p+1}, t_q)$ et $M_1(z_p, t_{q-1})$ sélectionnés au cours de l'étape 140, respectivement à partir des images $I(z_{p+1}, t_{q-1})$, $I(z_{p+1}, t_q)$ et $I(z_p, t_{q-1})$. Ces masques forment des masques de base pour la première cellule. On a également représenté, autour de chaque masque, une bounding box.

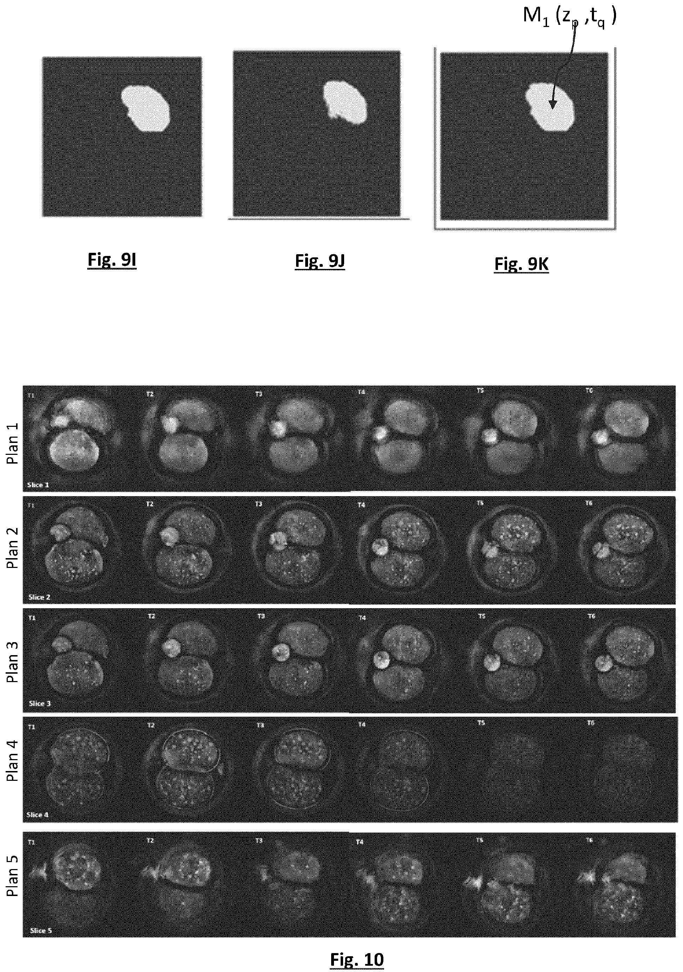

Chaque bounding box a été reportée sur l'image $I(z_p, t_q)$ .Il en résulte l'obtention de masques de segmentation représentés sur les figures 9I, 9J et 9K, ces masques étant obtenus sur la base d'un report, sur l'image $I(z, t)$, des bounding box correspondant respectivement aux masques $M_1(z_{p+1}, t_{q-1})$, $M_1(z_{p+1}, t_q)$ et $M_1(z_p, t_{q-1})$. Après filtrage, le masque retenu est celui représenté sur la figure 9K. Ce dernier est désigné $M_1(z_q, t_q)$ car il correspond au masque défini sur l'image $I(z_p, t_q)$ pour la première cellule (u = 1). Ce masque peut être utilisé pour prompter une segmentation d'une image de la pile d'images $P(t_q)$ ou une image, de préférence voisine, d'une autre pile d'images de la même plage temporelle Δt.

Suite à l'étape 150, on dispose, pour chaque pile d'images $P(t)$, et pour chaque cellule, d'un masque dit 3D, qui correspond à l'ensemble des masques définis, pour ladite cellule, sur chaque image $I(z, t)$ de la pile d'images.

Etape 160 : observation

Au cours de cette étape, les masques définis dans chaque image, pour chaque cellule, sont reportés sur les images de la pile d'images, afin de mettre en évidence chaque cellule. Par exemple, chaque cellule peut être contournée ou colorisée d'une certaine couleur, pour mieux permettre de la distinguer. Chaque cellule peut par exemple être colorisée d'une couleur différente, de façon à discriminer les cellules entre elles.

Par ailleurs, l'obtention de masques 3D pour chaque cellule permet d'accéder à des informations sur l'évolution morphologique de chaque cellule au cours du développement de l'échantillon cellulaire. Il s'agit par exemple de quantifier l'évolution de l'aire ou de la forme de chaque masque 3D.

La figure 10 montre, pour un embryon de souris comportant 4 cellules, une application de la segmentation 3D. On a représenté, sur la figure 10, différentes images dans des plans de coupe. Chaque ligne de la figure 10 correspond à un même plan de coupe et chaque colonne correspond au décours temporel.

Bien que décrite en lien avec un échantillon cellulaire de type embryon, notamment embryon non humain, l'invention peut être appliquée à la segmentation 3D d'autres types d'échantillons biologiques, par exemple des échantillons cellulaires de type organoïdes, ou organisme multicellulaire, ou d'autres types de structures biologiques 3D, comportant des objets biologiques dont le nombre et/ou la forme évoluent dans le temps.

**Revendications**

1. Procédé de segmentation 3D d'un échantillon, l'échantillon comportant au moins un objet biologique, l'échantillon se développant au cours du temps, de façon qu'au moins un objet biologique se divise ou change de forme ou de position au cours du temps, le procédé comportant :

   - a) en différents instants, acquisition d'une pile d'images *(P(t))* de l'échantillon, chaque image (*I*(*z, t*)) de la pile d'images représentant des images de l'échantillon, selon différents plans, à un instant, les instants définissant des plages temporelles, de façon qu'au cours de chaque plage temporelle ($\Delta t$), l'échantillon comporte un même nombre d'objets biologiques ($N(\Delta t)$) , au moins une pile d'images étant acquise dans chaque plage temporelle ;
   - b) segmentation d'au moins une image ($I(z_p,t_q)$) d'une pile d'images de façon à définir des masques sur ladite image, chaque masque étant délimité par contour fermé ;
   - c) sélection des masques définis lors de l'étape b) en fonction de critères de sélection prédéfinis, de façon que chaque masque sélectionné ($M_u$

($z_p,t_q$)) corresponde à un objet biologique, chaque masque sélectionné étant associé à une position qui correspond à une position du masque sur l'image segmentée;
   - d) réitération des étapes b) et c) sur une autre image d'une pile d'images de la même plage temporelle, jusqu'à obtenir au moins un masque sélectionné ($M_u(z_p,t_q)$) pour chaque objet biologique de l'échantillon ;
   - e) sélection d'une pile d'images, acquise durant une plage temporelle dite plage temporelle d'intérêt ;
   - f) pour au moins un objet biologique, à partir d'au moins un masque ($M_u(z_p,t_q)$), sélectionné dans la plage temporelle d'intérêt, et correspondant à l'objet biologique, segmentation de chaque image de la pile d'image sélectionnée dans l'étape e), de façon à déterminer, dans plusieurs d'images de ladite pile d'images, des masques correspondant à l'objet biologique, la segmentation étant guidée par la position du masque sélectionné ;
   - g) répétition des étapes e) à f) pour différentes piles d'images dans au moins une plage temporelle ;

les étapes b) à g) étant mises en œuvre par une unité de traitement.

2. Procédé selon la revendication 1, dans lequel les étapes e) et f) sont répétées pour chaque pile d'images de chaque plage temporelle.

3. Procédé selon l'une quelconque des revendications précédentes, dans lequel l'étape f) est répétée pour chaque objet biologique de l'échantillon, dans au moins une plage temporelle.

4. Procédé selon l'une quelconque des revendications précédentes, dans lequel le procédé comporte, préalablement à l'étape b), une obtention d'un nombre N($\Delta t$) d'objets biologiques contenus dans l'échantillon, au cours de chaque plage temporelle, N($\Delta t$) étant un entier supérieur ou égal à 1.

5. Procédé selon la revendication 4, comportant, préalablement à l'étape b) une détermination du nombre d'objets biologiques, dans au moins une plage temporelle, à partir d'au moins une pile d'image acquise durant la plage temporelle.

6. Procédé selon la revendication 5, dans lequel :

   - les instants d'acquisition sont répartis en différentes plages temporelles, à chaque plage temporelle correspondant un nombre d'objets biologiques dans l'échantillon ;
   - le procédé comporte, préalablement à l'étape b), une classification de chaque pile d'images, de façon à assigner chaque pile d'images à une desdites plages temporelles.

7. Procédé selon la revendication 6, comportant préalablement à l'étape b) :

- i) application d'un algorithme de réduction de la dimension à chaque pile d'images, de façon à assigner une coordonnée à chaque pile d'images dans un espace latent ;
- ii) dans l'espace latent, assignation de chaque coordonnée dans une classe ;
- iii) détermination du nombre d'objets biologique dans l'échantillon, pour chaque pile d'images, en fonction de la classe correspondant à la pile d'images.

8. Procédé selon la revendication 7, dans lequel l'étape i) comporte une utilisation d'un réseau de neurones encodeur, à partir de chaque pile d'images, pour obtenir un code correspondant à chaque pile d'image, l'algorithme de réduction de la dimension étant appliqué à partir du code.

9. Procédé selon la revendication 7, dans lequel l'étape i) comporte une formation d'une image représentative de chaque pile d'images, pour alimenter le réseau de neurones encodeur, l'image représentative pouvant être une image de type image de projection maximale établie pour chaque pile d'images.

10. Procédé selon l'une quelconque des revendications précédentes, dans lequel dans l'étape c), au moins un critère de sélection est choisi parmi :

- un critère morphologique du masque ;
- un recouvrement maximal entre deux masques sélectionnés.

11. Procédé selon l'une quelconque des revendications précédentes, dans lequel, l'étape c) comporte une détermination d'une propriété physique de l'échantillon dans une partie de l'image correspondant à chaque masque, le critère de sélection dépendant de la valeur de ladite propriété physique.

12. Procédé selon l'une quelconque des revendications précédentes, dans lequel :

- lors de l'étape a), deux piles d'images successivement acquises sont décalées d'un incrément temporel, deux images adjacentes d'une même pile d'images étant décalées d'un incrément spatial,;
- chaque image, sur laquelle au moins un masque a été sélectionné lors d'une étape c) est une image de référence pour l'objet biologique correspondant au masque ;
- l'étape f) comporte :

• f-i) sélection d'un objet biologique ;
• f-ii) sélection d'une image dans laquelle aucun masque n'a été défini pour l'objet biologique sélectionné, l'image sélectionnée comportant une coordonnée spatiale et une coordonnée temporelle ;
• f-iii) sélection d'un masque correspondant à l'objet biologique sélectionné dans une image de référence, pour l'objet biologique sélectionné dans la sous-étape f-i), voisine de l'image sélectionnée dans la sous-étape f-ii), l'image de référence voisine ayant des coordonnées respectivement spatiale et temporelle décalées respectivement d'un nombre d'incréments spatiaux et temporels inférieur à un seuil prédéterminé ;
• f-iv) report de la position du masque sélectionné dans la sous-étape f-iii) sur l'image sélectionnée dans la sous-étape f-ii) ;
• f-v) segmentation de l'image sélectionnée dans la sous-étape f-ii), en utilisant la position reportée dans la sous-étape f-iv), de façon à définir un masque pour l'objet biologique sélectionné dans la sous-étape f-i), la segmentation étant guidée par ladite position du masque.

13. Procédé selon la revendication 12, dans lequel suite à l'étape f-v), l'image sélectionnée dans la sous-étape f-ii) devient une image de référence pour l'objet biologique sélectionné dans la sous-étape f-i).

14. Dispositif d'observation d'un échantillon comportant :

- un système d'acquisition (1), configuré pour former une pile d'images de l'échantillon en différents instants, chaque pile d'images représentant l'échantillon à un instant, les instants définissant des plages temporelles, au cours desquelles l'échantillon comporte un même nombre d'objets biologiques, de façon qu'à chaque plage temporelle correspond au moins une pile d'images;
- une unité de traitement, configurée pour mettre en œuvre les étapes b) à g) d'un procédé selon l'une quelconque des revendications précédentes, à partir des piles d'images respectivement formées aux différents instants.

15. Support, pouvant être connecté à un ordinateur, comportant des instructions pour mettre en œuvre l'étape b) à g) d'un procédé selon l'une quelconque des revendications 1 à 13 à partir de piles d'images d'un échantillon.

**Fig. 1A**

**Fig. 1B**

**Fig. 2**

$P(t)$   $I(z,t)$

$I_{maxproj}(t)$

$C(t)$

$N(\Delta t)$

**Fig. 3A**

**Fig. 3B**

**Fig. 3C**

$$in \longrightarrow \boxed{Ext} \longrightarrow C(t) \longrightarrow \boxed{Recons} \longrightarrow out$$

**Fig. 4**

**Fig. 5A**

**Fig. 5D**

**Fig. 5B**

**Fig. 5E**

**Fig. 5C**

**Fig. 5F**

Fig. 6

Fig. 7

$I(z_p, t_q)$

**Fig. 8A**

**Fig. 8B**

$M_1(z_p, t_q)$

**Fig. 8C**

$I(z_{p+5}, t_q)$

**Fig. 8D**

**Fig. 8E**

$M_3(z_{p+5}, t_q)$ $M_2(z_{p+5}, t_q)$

$M_1(z_p, t_q)$

**Fig. 8F**

$$I(z_{p+5}, t_{q+1})$$

**Fig. 8G**

**Fig. 8H**

$M_3(z_{p+5}, t_q)$ $\quad$ $M_2(z_{p+5}, t_q)$ $\quad$ $M_1(z_p, t_q)$

$M_4(z_{p+5}, t_{q+1})$

**Fig. 8I**

$I(z_p, t_q)$

**Fig. 9A**

**Fig. 9B**

$I(z_{p+1}, t_{q-1})$ **Fig. 9C**

$M_1(z_{p+1}, t_{q-1})$

**Fig. 9F**

$I(z_{p+1}, t_q)$

**Fig. 9D**

$M_1(z_{p+1}, t_q)$

**Fig. 9G**

$I(z_p, t_{q-1})$ **Fig. 9E**

$M_1(z_p, t_{q-1})$

**Fig. 9H**

Fig. 9I          Fig. 9J          Fig. 9K

Fig. 10

Europäisches Patentamt
European Patent Office
Office européen des brevets

## RAPPORT DE RECHERCHE EUROPEENNE

Numéro de la demande

EP 25 17 9762

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | COPPERMAN JEREMY ET AL: "Morphodynamical cell state description via live-cell imaging trajectory embedding", COMMUNICATIONS BIOLOGY , vol. 6, no. 1 4 mai 2023 (2023-05-04), XP093191456, ISSN: 2399-3642, DOI: 10.1038/s42003-023-04837-8 Extrait de l'Internet: URL:https://www.nature.com/articles/s42003 -023-04837-8.pdf * le document en entier * ----- | 1-15 | INV. G06V20/69 G06T7/00 G06T7/11 G06T7/174 G06T7/194 |
| X | Zargari Abolfazl ET AL: "DeepSea: An efficient deep learning model for automated cell segmentation and tracking", bioRxiv, 10 mars 2021 (2021-03-10), pages 1-16, XP055795229, DOI: 10.1101/2021.03.10.434806 Extrait de l'Internet: URL:https://www.biorxiv.org/content/10.110 1/2021.03.10.434806v1.full.pdf [extrait le 2021-04-14] * le document en entier * ----- | 1-15 | **DOMAINES TECHNIQUES RECHERCHES (IPC)** G06V G06T |
| X | LEÓN JUAN ET AL: "Learning to segment mouse embryo cells", PROCEEDINGS OF SPIE; [PROCEEDINGS OF SPIE ISSN 0277-786X VOLUME 10524], SPIE, US, vol. 10572, 17 novembre 2017 (2017-11-17), pages 1057212-1057212, XP060095945, DOI: 10.1117/12.2285967 ISBN: 978-1-5106-1533-5 * le document en entier * ----- -/-- | 1-15 | |

Le présent rapport a été établi pour toutes les revendications

| Lieu de la recherche | Date d'achèvement de la recherche | Examinateur |
|---|---|---|
| La Haye | 17 septembre 2025 | Grigorescu, Cosmin |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
........................................................................................
& : membre de la même famille, document correspondant

EPO FORM 1503 03.82 (P04C02)

page 1 de 2

**RAPPORT DE RECHERCHE EUROPEENNE**

Europäisches
Patentamt
European
Patent Office
Office européen
des brevets

Numéro de la demande

EP 25 17 9762

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | KHAN AISHA ET AL: "A Linear Chain Markov Model for Detection and Localization of Cells in Early Stage Embryo Development", 2015 IEEE WINTER CONFERENCE ON APPLICATIONS OF COMPUTER VISION, IEEE, 5 janvier 2015 (2015-01-05), pages 526-533, XP032738171, DOI: 10.1109/WACV.2015.76 * le document en entier * ----- | 1-15 | |

DOMAINES TECHNIQUES
RECHERCHES (IPC)

Le présent rapport a été établi pour toutes les revendications

| Lieu de la recherche | Date d'achèvement de la recherche | Examinateur |
|---|---|---|
| La Haye | 17 septembre 2025 | Grigorescu, Cosmin |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

page 2 de 2

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 4207077 A

**Littérature non-brevet citée dans la description**

- **COPPERMAN J. et al.** Morphological cell state description via live-cell imaging trajectory embedding. *Communication Biology*, 04 May 2023, vol. 6 (1)
- **ZARGARI A. et al.** DeepSea : an efficient deep learning model for automated cell segmentation and tracking. *bioRxiv*, 10 March 2021
- **LEON J. et al.** Learning to segment mouse embryo cells. *proceedings of SPIE*, 17 November 2017, vol. 10572
- **CEN, J**. Segment Anything Model. *Segment Anything in 3D with NerFs*